# EUROPEAN PATENT APPLICATION

(11) **EP 4 740 947 A1**
(43) Date of publication of application: **13.05.2026**
(21) Application number: 24848331.5
(22) Date of filing: 01.08.2024
(51) Int. Cl.: A61K 31/505, A61K 31/506, A61K 31/437, A61K 31/5377, A61P 35/00, A61P 35/02

(54) **PHARMACEUTICAL COMBINATION OF EGFR KINASE INHIBITOR AND C-MET KINASE INHIBITOR FOR TREATING TUMOR DISEASES**

(30) Priority: 02.08.2023 CN 202310970721
(71) Applicant: Jiangsu AoSaiKang Pharmaceutical Co., Ltd., Jiangning Science Park, Jiangning Nanjing, Jiangsu 211112 (CN)
(72) Inventor: CHEN, Hongyu, Nanjing, Jiangsu 211112 (CN); SONG, Tingting, Nanjing, Jiangsu 211112 (CN); XU, Zheng, Nanjing, Jiangsu 211112 (CN); SUN, Jiye, Nanjing, Jiangsu 211112 (CN); ZONG, Zaiwei, Nanjing, Jiangsu 211112 (CN); HAN, Luwei, Nanjing, Jiangsu 211112 (CN); CHEN, Jing, Nanjing, Jiangsu 211112 (CN)
(74) Representative: karo IP
(86) International application number: PCT/CN2024/109080
(87) International publication number: WO 2025/026376

(57) **Abstract**

The present invention relates to a drug combination of an EGFR kinase inhibitor and a c-Met kinase inhibitor, and use thereof in the preparation of a drug for treating tumor diseases. Specifically, a drug combination of a compound of Formula (I) or a pharmaceutically acceptable salt thereof and a compound of Formula (II) or a pharmaceutically acceptable salt thereof is provided. The drug combination shows a stronger anti-tumor activity, has no obvious toxic side effects, and has wide prospects of application.

## Description

### TECHNICAL FIELD

The present invention belongs to the field of medicine, and specifically relates to a drug combination of an EGFR kinase inhibitor and a c-Met kinase inhibitor, and use thereof in the preparation of a drug for treating tumor diseases.

### BACKGROUND

Lung cancer is the most common malignant tumor in China and even in the world. The latest global cancer data released by the International Agency for Research on Cancer (IARC) of the World Health Organization in 2020 shows that the number of newly found patients with lung cancer in the world is 2.2 million, with the incidence ranked the 2nd place and the mortality ranked the 1st place. In China, the incidence and mortality of lung cancer are ranked the 1st place out those of all malignant tumors, and the incidence and mortality increase with age. Histopathologically, lung cancer can be classified into non-small cell lung cancer (NSCLC) and small cell lung cancer (SCLC), where NSCLC accounts for about 85% of all lung cancers, and about 75% of patients with NSCLC are in the middle and advanced stage at the time of diagnosis, with low 5-year survival rate and poor prognosis. For unresectable middle-stage and advanced lung cancer, although the traditional radiotherapy and chemotherapy have made some progress, the treatment efficiency is still low, and the advanced or metastatic lung cancer is still a fatal disease with a large number of unmet medical needs.

Epidermal growth factor receptor (EGFR) gene is one of the most common driver genes in NSCLC, and about 50% of patients with NSCLC in China have EGFR gene mutation (J Thorac Oncol, 2014, 9(9):e70). EGFR tyrosine kinase inhibitors (TKIs), as the most commonly used targeted drugs, have made a breakthrough, and EGFR kinase inhibitors have become the standard first-line treatment for patients with EGFR mutation-positive advanced NSCLC.

Although EGFR kinase inhibitors have become the standard first-line treatment for patients with EGFR mutation induced NSCLC, the patients have developed drug resistance after 9-14 months of treatment, due to the mechanism including new mutation or amplification of EGFR or other genes. The existing research data show that the amplification of mesenchymal epithelial transition factor (MET) gene may be one of the causes leading to acquired resistance in patients to EGFR kinase inhibitors. MET alteration occurs in some patients with NSCLC that are EGFR mutation positive and have acquired resistance to EGFR kinase inhibitors. Therefore, the combination of an EGFR kinase inhibitor and a c-MET kinase inhibitor has potential application value in the treatment of NSCLC patients with drug resistance caused by MET gene amplification or MET protein overexpression.

WO2016/082713A1 discloses a class of 2-aminopyrimidine compounds, which can potently inhibit the activity of a kinase having the drug resistance inducing mutation EGFR T790M and sensitive EGFR mutation, and show significant anti-tumor activity in vitro and in vivo for tumors driven by EGFR mutation. The compound N-((5-((5-chloro-4-((naphthalen-2-yl)amino)) pyrimidin-2-yl)amino)-2-((N-methyl-N-dimethylaminoethyl)amino)-4-methoxyphenyl) acrylamide has a structure of Formula (I):

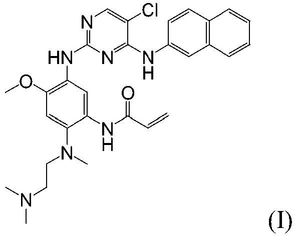

WO2020/156453A1 discloses a class of tricyclic compounds containing a pyrimidinyl group, which can effectively and selectively inhibit the activity of c-MET kinase, and can be used to treat diseases mediated by MET alteration, and specifically discloses compounds of Formula (II):

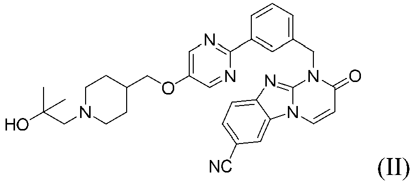

### SUMMARY

In a first aspect of the present invention, a drug combination for treating tumor diseases is provided. The drug combination comprises (i) an EGFR kinase inhibitor; and (ii) a c-Met kinase inhibitor.

In some embodiments, the EGFR kinase inhibitor is selected from a compound of Formula (I) or a pharmaceutically acceptable salt thereof, and Gefitinib, Erlotinib, Icotinib, Lapatinib, Afatinib, Dacomitinib, Neratinib, Osimertinib, Almonertinib, Rociletinib, Furmonertinib, Rociletinib, Abivertinib, Befotertinib, Lazertinib, CK-101, ASP8273, nazartinib or a pharmaceutically acceptable salt thereof.

In some embodiments, the c-Met kinase inhibitor is selected from a compound of Formula (II) or a pharmaceutically acceptable salt thereof, and Crizotinib, Cabozantinib, Capmatinib, Tepotinib, Volitinib, Gumarontinib, Sitravatinib, EMD-1214063, Elzovantinib, Bozitinib, XL-092, AL-2846, TAS-115, AMG-337, HS-10241, TQ-B3139, ABN-401or a pharmaceutically acceptable salt thereof.

In an exemplary embodiment, the EGFR kinase inhibitor is selected from a compound of Formula (I) or a pharmaceutically acceptable salt thereof, and the c-Met kinase inhibitor is selected from a compound of Formula (II) or a pharmaceutically acceptable salt.

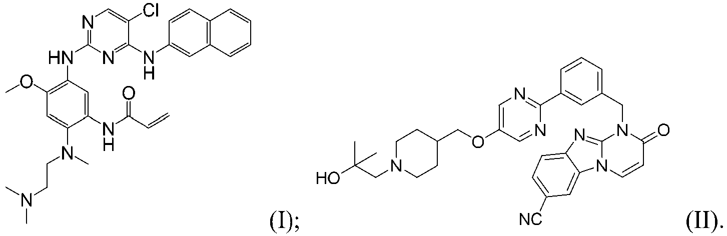

In a second aspect of the present invention, use of a combination of the compound of Formula (I) or a pharmaceutically acceptable salt thereof and the compound of Formula (II) or a pharmaceutically acceptable salt thereof in the preparation of a drug for treating tumor diseases is provided.

In some embodiments, the tumor diseases are selected from breast cancer, ovarian cancer, prostate cancer, melanoma, brain tumor, esophageal carcinoma, gastric cancer, liver cancer, pancreatic cancer, colorectal cancer, lung cancer, kidney cancer, skin cancer, glioblastoma, neuroblastoma, sarcoma, liposarcoma, osteochondroma, osteoma, osteosarcoma, seminoma, testicular tumor, cervical cancer, head and neck tumor, multiple myeloma, malignant lymphoma, polycythemia vera, leukemia, thyroid neoplasm, ureteral tumor, bladder cancer, gallbladder cancer, bile duct carcinoma or chorionepithilioma, preferably lung cancer, and further preferably non-small cell lung cancer.

In some preferred embodiments, the non-small cell lung cancer is selected from squamous cell carcinoma or non-squamous cell carcinoma, and preferably non-squamous cell carcinoma, where the non-squamous cell carcinoma can be adenocarcinoma, large cell carcinoma and other subtypes of cell carcinoma.

In some preferred embodiment, the tumor diseases are tumor diseases in patients resistant to EGFR kinase inhibitors.

In some preferred embodiments, the tumor diseases are tumor diseases associated with EGFR mutation and/or MET alteration; and preferably tumor diseases associated with one or more gene mutations selected from EGFR 19del, MET alteration, EGFR L858R, and EGFR T790M.

In some preferred embodiments, the tumor diseases are lung cancers in patients resistant to EGFR kinase inhibitors.

In some preferred embodiments, the tumor diseases are lung cancers associated with EGFR mutation and/or MET alteration, and preferably lung cancers associated with one or more gene mutations selected from EGFR 19del, MET alteration, EGFR L858R, and EGFR T790M.

In some preferred embodiments, the tumor diseases are non-small cell lung cancers in patients resistant to EGFR kinase inhibitors.

In some preferred embodiments, the tumor diseases are non-small cell lung cancers associated with EGFR mutation and/or MET alteration, and further preferably non-small cell lung cancers associated with one or more gene mutations selected from EGFR 19del, MET alteration, EGFR L858R, and EGFR T790M.

In an exemplary embodiment, the tumor diseases are non-small cell lung cancer that has disease progression after treatment with EGFR kinase inhibitors previously and is associated with MET alteration.

In some embodiments, the single dosage of the compound of Formula (I) or the pharmaceutically acceptable salt thereof is 40-320 mg, preferably 60-240 mg, and further preferably 80-160 mg.

In some embodiments, the frequency of administration of the compound of Formula (I) or the pharmaceutically acceptable salt thereof is once a day, twice a day, three times a day or once every two days.

In some embodiments, the single dosage of the compound of Formula (II) or the pharmaceutically acceptable salt thereof is 40-640 mg, preferably 40-320 mg, and further preferably 60-320 mg.

In some embodiments, the frequency of administration of the compound of Formula (II) or the pharmaceutically acceptable salt thereof is once a day, twice a day, three times a day or once every two days.

In some embodiments, the compound of Formula (I) or the pharmaceutically acceptable salt thereof and the compound of Formula (II) or the pharmaceutically acceptable salt thereof can be administered simultaneously or at different times.

In a third aspect of the present invention, a pharmaceutical preparation is further provided, which comprises the drug combination according to the first aspect of the present invention and one or more pharmaceutically acceptable carriers.

In the present invention, the term "pharmaceutically acceptable salt" means that when the compound contains a relatively acidic functional group, a base addition salt can be obtained by contacting such a compound with a sufficient amount of a base in a pure solution or a suitable inert solvent; or when the compound contains a relatively basic functional group, an acid addition salt can be obtained by contacting such a compound with a sufficient amount of an acid in a pure solution or a suitable inert solvent. The pharmaceutically acceptable base addition salt includes a sodium, potassium, calcium, ammonium, organic amine or magnesium salt or similar salts. Examples of the pharmaceutically acceptable acid addition salt include salts with inorganic acids, including, for example, hydrochloric acid, hydrobromic acid, nitric acid, carbonic acid, bicarbonate, phosphoric acid, dibasic phosphate, monobasic phosphate, sulfuric acid, bisulfate, hydroiodic acid, phosphorous acid, and the like; salts with organic acids, including, for example, acetic acid, propionic acid, isobutyric acid, maleic acid, malonic acid, benzoic acid, succinic acid, octanedioic acid, fumaric acid, lactic acid, mandelic acid, phthalic acid, benzenesulfonic acid, p-toluenesulfonic acid, citric acid, tartaric acid, methanesulfonic acid, and the like; and salts of amino acids (such as arginine) and salts of organic acids such as glucuronic acid.

In the present invention, the term "combination" of drugs includes a combination of drugs at fixed dosages (in a single dosage form), combination package of drugs for combined administration, and also combination therapy of drugs. In terms of combination package of drugs or combination therapy of drugs, each drug can be administered simultaneously or separately at a certain time interval. In the present invention, the term "combination of drugs at fixed dosages" refers to a single dosage form prepared for delivering a certain amount of two or more drugs to a patient; and examples include compound preparations and composite preparations. In the present invention, the term "compound preparation" or "composite preparation" refers to a medicine consisting of several drug ingredients. The "compound preparation" refers to a preparation made by mixing several drugs of different types; and the "composite preparation" refers to a preparation comprising several drugs of the same type, but other types of drugs may also optionally be present. In the present invention, the term "combination package of drugs" refers to a package consisting of two or more pharmaceutical preparations with separate indications, usage and dosage. In the present invention, the term "combination therapy" of drugs refers to the administration of two or more drugs with independent indications, usage and dosage to a patient to treat related diseases.

In the present invention, the term "EGFR 19del" means deletion of exon 19 of EGFR; the term "EGFR L858R" means that the amino acid at position 858 of the EGFR protein is mutated from L to R; and the term "EGFR T790M" means that the amino acid at position 790 of the EGFR protein is mutated from T to M. "EGFR 19del" and "EGFR L858R" are common mutations of EGFR, which are sensitive to the 1st-3rd generation of EGFR kinase inhibitors.

In the present invention, the term "MET alteration" includes exon 14 skipping mutation of MET gene (MET 14 skipping mutation), MET gene amplification, MET gene point mutation (mainly mutations in kinase region), MET gene fusion and MET protein overexpression. The incidence of MET 14 skipping mutation in patients with NSCLC is 0.9%-4.0%; the incidence of primary MET gene amplification in patients with NSCLC is 1%-5%, the incidence of secondary MET gene amplification is 5%-50% in patients resistant to EGFR kinase inhibitors and about 13% in patients resistant to anaplastic lymphoma kinase inhibitors, and the incidence of MET protein overexpression in patients with NSCLC is 13.7%-63.7%. See Chinese Journal of Pathology, 2022, 51(11): 1094-1103.

### Beneficial effects of the present invention

The present invention provides a drug combination of an EGFR kinase inhibitor and a c-Met kinase inhibitor, and use thereof in the preparation of a drug for treating tumor diseases. Specifically, a drug combination of a compound of Formula (I) or a pharmaceutically acceptable salt thereof and a compound of Formula (II) or a pharmaceutically acceptable salt thereof is provided. The drug combination shows a stronger anti-tumor activity, has no obvious toxic side effects, and has wide prospects of application.

### BRIEF DESCRIPTION OF THE DRAWINGS

Fig. 1 shows a weight change curve of mice in Example 1 during treatment.
Fig. 2 shows a tumor volume change curve in Example 1.
Fig. 3 shows a tumor volume change curve in Example 2.
Fig. 4 shows a picture of the tumor (day 21) after the test drug is administered to a patient derived xenograft tumor model established with LD1-0025-200662 human lung cancer tissue in NU/NU mice in Example 2, in which CR indicates the complete regression (remission) of the tumor, and G1-G7 correspond to the grouping and administration information in Table 9 in Example 2.
Fig. 5 shows a weight change curve of mice in Example 2 during treatment.

### DETAILED DESCRIPTION

The present invention is further described below in conjunction with specific examples. It is to be understood that these examples are merely illustrative of the present invention and are not intended to limit the scope of the present invention. Equivalent replacements or modifications made to the present invention also fall within the protection scope of the present invention. For experimental methods where no specific conditions are given in the following examples, conventional conditions or conditions recommended by the manufacturer are followed.

### Example 1: Efficacy study of test drugs in xenograft model established with engineered cell line NCI-H1975-HGF in immunodeficient mice

### 1. Experimental purpose

The efficacy of the test drugs in a xenograft model established with the engineered cell line **NCI-H1975-HGF** in immunodeficient mice was verified.

### 2. Experimental materials

### 2.1 Cells

The cell source information was shown in Table 1.

**Table 1. Cell information**

| **Cell name** | **Source** | **Cancer type** | **Passage** | **Cell amount needed** |
|---|---|---|---|---|
| NCI-H1975-HGF | KYBC | Engineered cell line | 15^{th}-25^{th} passages | 1.5*10⁷ |

### 2.2 Test animals

B-NDG mice, female, age: 8 weeks, weight: 18-22 g, purchased from Biocytogen.

### 2.3 Test drugs

Vehicle: 20% solutol/80% 100 mM sodium acetate buffer pH 5.5;
ASK-MET: hydrochloride of the compound of Formula (II), prepared with 20% solutol/80% 100 mM sodium acetate buffer pH 5.5, concentration: 0.2 mg/mL;
ASK-EGFR: compound of Formula (I), prepared with 0.5% sodium carboxymethyl cellulose solution (0.5%CMC-Na), concentration: 2 mg/mL.
Solutol: polyethylene glycol-15 hydroxystearate;
100 mM sodium acetate buffer pH 5.5;
20% solutol/80% 100 mM sodium acetate buffer pH 5.5 means that the volume ratio of solutol to 100 mM sodium acetate buffer pH 5.5 in the drug solution was 20:80, the same below.

### 3. Experimental method:

1) NCI-H1975-HGF was resuscitated and cultured in vitro to obtain 1.5*10⁷ cells.
2) 15 8-10-week-old immunodeficient mice were adaptively raised for 1 week, and weighed.
3) The inoculation conditions were shown in Table 2.

**Table 2. Inoculation information**

| **Animal breed** | **Number of animals** | **Cell type inoculated** | **Site of inoculation** | **Cell amount inoculated** | **Volume of cell suspension inoculated (mL)** | **Total cell amount needed** |
|---|---|---|---|---|---|---|
| B-NDG | 15 | NCI-H1975-HGF | S. C. | 1 *10⁶ | 0.1mL | 1.5 *10⁷ |

| | | | | | | |
|---|---|---|---|---|---|---|
| Note: s.c.: subcutaneous injection. | | | | | | |

4) After inoculation, the tumor volume and weight were measured twice a week. When the average tumor volume reached about 100 mm³, the animals were randomly divided into 4 groups according to the tumor volume and weight, each group having 3 animals. The animals were immediately administered after grouping. The day of administration was regarded as day 0. The administration and grouping information were shown in Table 3.

**Table 3. Grouping and administration information**

| **Grou p** | **Numbe r of animals** | **Treatment method** | **Administratio n Dosage (mg/kg)** | **Route of administratio n** | **Amount administere d, µL/g** | **Administratio n frequency** |
|---|---|---|---|---|---|---|
| G1 | 3 | Vehicle | NA | *p.o.* | 10 | QD×15 |
| G2 | 3 | ASK-MET | 2 | *p.o.* | 10 | QD×15 |
| G3 | 3 | ASK-MET + ASK-EGF R | 2+20 | *p.o.* | 10 | QD×15 |
| G4 | 3 | ASK-EGF R | 20 | *p.o.* | 10 | QD×15 |

| | | | | | | |
|---|---|---|---|---|---|---|
| Note: *p.o.:* oral administration; QD: once a day. | | | | | | |

5) administration on Day 0.
6) After the administration was completed, the test was ended.

### 4. Observation and data collection

### 4.1 Daily observation

After inoculation, the disease attack and death of animals were observed every working day. Daily observation includes tumor growth and the effects of drugs on test animals, such as changes in activity, changes in food and water intake, weight loss, appearance changes in hair and eyes, death and other clinical symptoms.

### 4.2 Body weight monitoring

After inoculation and before grouping, the weight of test animals was measured once a week. After grouping, the weight of the test animals was measured twice a week.

### 4.3 Tumor volume measurement

After inoculation and before grouping, when the tumor was visible, the tumor volume of the test animals was measured once a week. After grouping, the tumor volume of animals in the experiment was measured twice a week. The tumor volume was measured by a two-way measurement method. The major and minor diameter of the tumor were measured by vernier caliper, and then the tumor volume was calculated by the formula TV = 1/2 × a × b², where a is the major diameter of the tumor and b is the minor diameter of the tumor.

### 5. Analysis method of experimental endpoint data

At the end of the experiment, the following analysis methods were used for data analysis (Table 4).

**Table 4. Data analysis methods**

| **Experimental endpoint** | **Description/formula** |
|---|---|
| ΔT/ΔC(%) | ΔT/ΔC(%) = (mean(T)-mean(T0)) / (mean(C)-mean(C0)) * 100% |
| | T - tumor volume in the treatment group |
| | T0 - initial tumor volume in the treatment group |
| | C - tumor volume in the control group |
| | C0 - initial tumor volume in the control group |
| TGI (%) | TGI(%) =(((mean(C)-mean(C0))- (mean(T)-mean(T0))) / (mean(C)-mean(C0)) * 100% |
| | T - tumor volume in the treatment group |
| | T0 - initial tumor volume in the treatment group |
| | C - tumor volume in the control group |
| | C0 - initial tumor volume in the control group |

### 6. Statistical analysis of data

For an experiment having two groups, T-Test analysis method is used; and for comparison of three or more groups, One-Way ANOVA method is used for analysis. For the potential synergistic effect, Two-Way ANOVA is used. There is a significant difference when p value is less than 0.05.

### 7. Experimental results

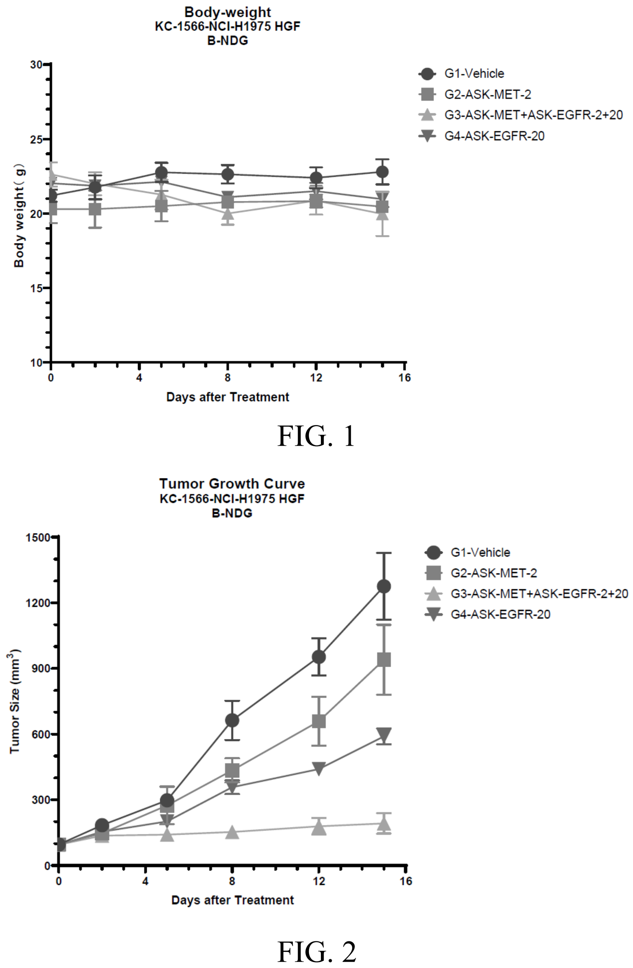

In this experiment, the test mice have no obvious symptoms, and the overall weight does not change significantly. In the treatment group, the weight of mice decreases only in the combined treatment group, but the weight of an individual mouse decreases by no more than 20%. The experiment is ended on Day 17, and the data of Day 15 is used for analysis. The weight data, tumor volume data and tumor growth inhibition in each group are shown in FIGs. 1 and 2, and Tables 5, 6 and 7.

**Table 5. Weight change in each group**

| **Weight (g)** | | | | | | |
|---|---|---|---|---|---|---|
| Days | 0 | 2 | 5 | 8 | 12 | 15 |
| G1 | 21.2+40.4 | 21.8±0.8 | 22.8±0.6 | 22.6±0.6 | 22.4±0.7 | 22.8±0.9 |
| G2 | 20.3±1.0 | 20.3±1.3 | 20.5±1.0 | 20.8±0.3 | 20.8±0.4 | 20.5±0.4 |
| G3 | 22.6±0.8 | 22.0±0.8 | 21.3±1.0 | 20.0±0.8 | 20.9±0.9 | 20.0±1.5 |
| G4 | 22.0±0.1 | 21.9±0.2 | 22.1±0.1 | 21.1±0.3 | 21.5±0.6 | 21.0±0.5 |

**Table 6. Average tumor volume data of each group in the experiment**

| **NCI-H1975 HGF tumor volume (mm³)** | | | | | | |
|---|---|---|---|---|---|---|
| Days | 0 | 2 | 5 | 8 | 12 | 15 |
| G1 | 95.79 ±6.47 | 184.32 ±15.00 | 297.97 ±27.81 | 663.42 ±89.72 | 952.92 ±85.69 | 1275.47 ±152.44 |
| G2 | 96.28 ±7.10 | 147.46 ±23.06 | 274.61 ±85.69 | 434.60 ±55.60 | 658.86 ±111.99 | 940.03 ±159.86 |
| G3 | 95.32 ±7.55 | 136.72 ±19.37 | 140.98 ±20.46 | 153.32 ±24.59 | 179.22 ±37.72 | 192.22 ±45.80 |
| G4 | 94.47 ±9.54 | 154.47 ±24.23 | 201.41 ±24.69 | 357.90 ±31.91 | 440.56 ±16.92 | 589.86 ±36.92 |

**Table 7. Anti-tumor effects of test drugs in each group**

| **Group** | **Tumor volume at day 0 (mm³)^{a}** | **Tumor volume at day 15 (mm³)^{a}** | **TGI (%)** | **ΔT/ΔC (%)** | **P value** |
|---|---|---|---|---|---|
| G1 | 95.79±6.47 | 1275.47±152.44 | NA | NA | NA |
| G2 | 96.28±7.10 | 940.03±159.86 | 28.48% | 71.52% | 0.072 |
| G3 | 95.32±7.55 | 192.22±45.80 | 91.79% | 8.21% | <0.001 |
| G4 | 94.47±9.54 | 589.86±36.92 | 58.01% | 41.99% | 0.003 |

| | | | | | |
|---|---|---|---|---|---|
| Note: a: mean ± standard error; P value: treatment group (G2-G4) vs solvent control group (G1), statistically calculated by One-Way ANOVA. | | | | | |

### 8. Experimental conclusion and discussion

In group G2, on day 15 of administration of ASK-MET-2 mg/kg, *p.o.* QD, the average tumor volume is 940.03±159.86 mm³, which is less than the average tumor volume of the control group (1275.47±152.44 mm³); and in the group TGI (%)=28.48%, and p >0.05 compared with the control group.

In group G3, on day 15 of administration of ASK-MET+ASK-EGFR-2+20 mg/kg, *p.o.* QD, the average tumor volume is 192.22±45.80 mm3, which is less than the average tumor volume of the control group (1275.47±152.44 mm³); and in the group TGI (%)=91.79%, and p<0.05 compared with the control group.

In group G4, on day 15 of administration of ASK-EGFR-20 mg/kg, *p.o.* QD, the average tumor volume is 589.86±36.92 mm³, which is less than the average tumor volume of the control group (1275.47±152.44 mm³); and in the group TGI (%)=58.01%, and p<0.05 compared with the control group.

In summary, ASK-MET+ASK-EGFR-2+20 mg/kg (QD) in the combined treatment group has a high efficacy, and the combination of the two has a significant synergistic effect.

### Example 2: Efficacy study of test drugs in patient derived xenograft (PDX) tumor model established with LD1-0025-200662 human lung cancer tissue

### 1. Experimental purpose

NU/NU mice were subcutaneously inoculated with human lung cancer tissue LD1-0025-200662 to establish an in-vivo xenograft tumor model, and then the efficacy of the test drugs ASK-MET alone, ASK-EGFR alone, ASK-MET+ASK-EGFR in combination and RefT+ ASK-EGFR in combination in the model were evaluated.

### 2. Experimental materials

### 2.1 In-vivo xenograft tumor model

LD1-0025-200662 (see Table 8), a patient derived human lung cancer tissue, was sub-cultured to FP3+3 passages for this efficacy test.

**Table 8. Xenograft tumor model**

| **Model No.** | **Patient sex** | **Patient age** | **Pathological information** | **Mutation information** |
|---|---|---|---|---|
| LD1-0025-200662 | Male | 57 | Lung cancer-poorly differentiated adenocarcinoma | EGFR19 exon deletion, c-MET gene amplification |

### 2.2 Test animals

NU/NU mice, female, age: 42-62 days, weight: 18-22 g, purchased from Zhejiang Vital River Laboratory Animal Technology Co., Ltd. (Production license: SCXK(Zhe)2019-0001).

### 2.3 Test drugs

Vehicle: 20% solutol/80% 100 mM sodium acetate buffer pH 5.5;
ASK-MET: hydrochloride of the compound of Formula (II), prepared with 20% solutol/80% 100 mM sodium acetate buffer pH 5.5, concentration: 0.1 mg/mL, 0.3 mg/mL;
ASK-EGFR: compound of Formula (I), prepared with 0.5% sodium carboxymethyl cellulose solution (0.5%CMC-Na), concentration: 2 mg/mL;
RefT: prepared with 20% solutol/80% 100 mM sodium acetate buffer pH 5.5, concentration: 0.3 mg/mL; RefT is a small-molecule MET kinase inhibitor approved by USFDA.

### 3. Experimental method:

Immunodeficient mice were subcutaneously inoculated with LD1-0025-200662 tumor tissue, and observed continuously till tumor formation. When the tumor volume reached about 1000 mm³, the tumor tissue was peeled off and cut into tumor tissue with a size of about 3 mm × 3 mm × 3 mm (about 50-90 mg), and inoculated subcutaneously in NU/NU mice. The inoculated mice were observed and the growth of tumor was monitored. When the average tumor volume of tumor-bearing mice reached 193.40 mm³, the mice were randomly divided into groups, administered and observed. The tumor volume and weight of all tumor-bearing mice were measured before grouping, and the tumor-bearing mice were randomly grouped according to the measured tumor volume. According to the principle of random block design, the mice were first divided into blocks according to the tumor volume, and then the mice in each block were randomly assigned to each treatment group. The day of grouping was day 0, and the specific grouping and administration information were shown in Table 9 below.

**Table 9. Administration and treatment**

| **Group** | **Treatment group** | **Number of animals** | **Dosage (mg/kg)** | **Administration regimen** | **Route of administration** |
|---|---|---|---|---|---|
| G1 | Vehicle | 8 | -- | QD × 21 | *p.o.* |
| G2 | ASK-EGFR | 8 | 20 | QD×21 | *p.o.* |
| G3 | ASK-MET | 8 | 1 | QD × (D0- D7) | *p.o.* |
| G4 | ASK-MET | 8 | 3 | QD× (D0- D7) | *p.o.* |
| G5 | ASK-EGFR | 8 | 20 | QD × 21 | *p.o.* |
| | ASK-MET | | 1 | QD × (D0- D7) | *p.o.* |
| G6 | ASK-EGFR | 8 | 20 | QD × 21 | *p.o.* |
| | ASK-MET | | 3 | QD × (D0- D7) | *p.o.* |
| G7 | ASK-EGFR | 8 | 20 | QD × 21 | *p.o.* |
| | RefT | | 3 | QD × (D0- D7) | *p.o.* |

| | | | | | |
|---|---|---|---|---|---|
| Note: *p.o.:* oral administration; QD: once a day. Volume of administration: The volume of administration was adjusted according to the weight of tumor-bearing mice (0.1 mL/10 g). | | | | | |

### 4. Experimental observation

During the whole experiment, the use and observation of test animals were carried out in accordance with the relevant regulations of Guide for the Care and Use of Laboratory Animals of AAALAC. After the test animals were inoculated with the tumor tissue, they were observed every day and the disease attack and death were recorded. During the routine experiment, all the test animals were monitored and recorded for behavior, food intake, water intake, weight change, hair luster and other abnormal conditions.

### 5. Evaluation indicators

The growth inhibition or complete cure ability of the test drugs on LD1-0025-200662 human lung cancer xenograft tumor in vivo were mainly tested.

**5.1** Measurement of tumor volume and weight of tumor-bearing mice: The measurement was made twice a week by using a vernier caliper, and the tumor volume was calculated by a formula V = 0.5 × a × b², where a and b were respectively the major and minor diameters of the tumor.

**5.2** Tumor growth inhibition TGI (%)= [1-(Tᵢ-T₀) / (Vᵢ-V₀)] × 100, where Tᵢ-T₀>0; TGI (%) = [1-(Tᵢ-T₀)/ T₀] × 100, where Tᵢ-T₀<0. T₀ was the average tumor volume at the first administration in the treatment group, Tᵢ was the average tumor volume at a certain measurement after administration in the treatment group, V₀ was the average tumor volume at the first administration in the vehicle control group, and Vᵢ was the average tumor volume at a certain measurement after administration in the vehicle control group.

**5.3** Relative tumor proliferation rate T/C (%): The calculation formula is as follows: T/C % = T_{RTV} / C_{RTV} × 100 % (T_{RTV}: RTV of the treatment group; C_{RTV}: RTV of the negative control group). According to the results of tumor measurement, the relative tumor volume (RTV) was calculated by a formula RTV = Vt / V₀, where V₀ was the average tumor volume measured at the time of grouping for administration (that is, d₀), Vt was the average tumor volume measured at a certain measurement, and T_{RTV} and C_{RTV} data of the same day was used.

**5.4** Synergistic effect in combined treatment group: Jin's formula Q=E(a+b)/[Ea+Eb-Ea × Eb], where Ea was TGI of a drug alone, and Q value>1 reflected that drug combination has a synergistic effect.

**5.5** After the experiment, the tumor was stripped, weighed and photographed. T/C_{weight}%= T_{weight} /V_{weight} × 100%, where T_{weight} was the average tumor weight of the compound group at the end of the experiment, and V_{weight} was the average tumor weight of the vehicle control group at the end of the experiment.

**5.6** The weight of tumor-bearing mice was measured twice a week, and the change rate of weight gain of mice after administration was calculated: RCBW(%) = (BWᵢ - BW₀) / BW₀ × 100, where BWᵢ was the average weight at a certain measurement after administration, and BW₀ was the average weight at the first administration.

### 6. Statistical analysis of data

All data is expressed as Mean±SEM. One-Way ANOVA LSD(L) test is used to compare whether there are significant differences between the tumor volumes and tumor weights of various groups. * *P<* 0.05 means that there is a significant difference.

### 7. Experimental results

### 7.1 Tumor volume and weight

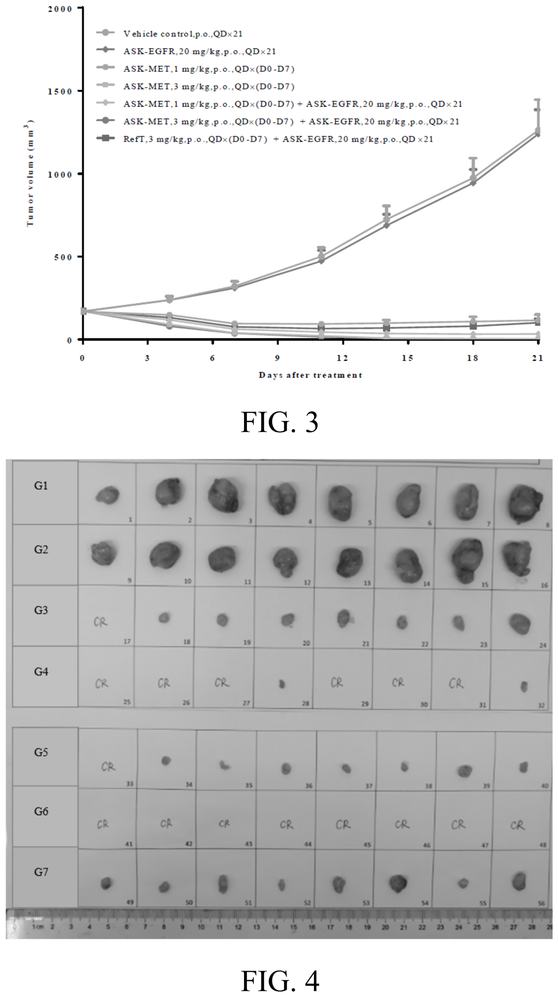

The purpose of this experiment is to subcutaneously inoculate LD1-0025-200662 human lung cancer tissue in NU/NU mice, to establish a PDX tumor animal model with LD1-0025-200662 in vivo, and then the anti-tumor effect of the test drugs in this tumor model is evaluated. The tumor volume changes, tumor pictures and statistical analysis results of the treatment group after the test drugs are administered in the PDX animal model established with LD1-0025-200662 human lung cancer tissue are shown in FIG. 3, 4, and Tables 10, 11 and 12.

The administration period of this efficacy test is 21 days, and then the whole efficacy test is ended. On day 21, the average tumor volumes of tumor-bearing mice in the vehicle control group, ASK-EGFR 20 mg/kg treatment group, ASK-MET_1 mg/kg treatment group, ASK-MET_3 mg/kg treatment group, ASK-MET_1 mg/kg+ASK-EGFR_20 mg/kg treatment group, ASK-MET_3 mg/kg+ASK-EGFR_20 mg/kg treatment group, and RefT_3 mg/kg+ASK-EGFR_20 mg/kg treatment group are were respectively 1261.93±184.86 mm³, 1239.58±146.6 mm³, 116.01±35.11 mm³, 3.54±2.35 mm³, 32.67±11.56 mm³, 0.00±0.00 mm³ and 100.67±24.39 mm³. Compared with the vehicle control group, the tumor growth inhibition rates TGI (%) in the ASK-EGFR_20 mg/kg treatment group, the tumor growth inhibition rate TGI (%) in the ASK-MET_1 mg/kg treatment group, the tumor growth inhibition rate TGI (%) in the ASK-MET_3 mg/kg treatment group, ASK-MET_1 mg/kg+ASK-EGFR_20 mg/kg treatment group, ASK-MET_3 mg/kg+ASK-EGFR_20 mg/kg treatment group and RefT_3 mg/kg+ASK-EGFR_20 mg/kg treatment group are respectively 1.96%, 131.27%, 197.93%, 180.72%, 200.00% and 140.79%. According to the above analysis results, except for the ASK-EGFR_20 mg/kg treatment group, all other treatment groups show statistically significant inhibitory effect on tumor growth (P<0.001, and T/C%<40%). In addition, compared with the treatment group with a single drug alone, the ASK-MET_1 mg/kg+ASK-EGFR_20 mg/kg treatment group, ASK-MET_3 mg/kg+ASK-EGFR_20 mg/kg treatment group and RefT_3 mg/kg+ASK-EGFR_20 mg/kg treatment group all show a more significant inhibitory effect on tumor growth (P<0.05), with an obvious synergistic effect (Q>1).

On the day of the end of the experiment (day 21), the rates of complete tumor regression of the tumor-bearing mice in the vehicle control group, ASK-EGFR_20 mg/kg treatment group, ASK-MET 1 mg/kg treatment group, ASK-MET_3 mg/kg treatment group, ASK-MET_1 mg/kg+ASK-EGFR_20 mg/kg treatment group, ASK-MET 3 mg/kg+ASK-EGFR_20 mg/kg treatment group and RefT_3 mg/kg+ASK-EGFR_20 mg/kg treatment group are observed by naked eyes to be 0.00%, 0.00%, 12.5%, 75%, 12.5%, 100% and 0.00% respectively. Then, all tumor bearing mice are euthanized, and the subcutaneous xenograft tumors of the tumor-bearing mice are stripped and weighed. The results of tumor weight and tumor volume are consistent.

**Table 10. Efficacy study of test drugs on human lung cancer LD1-0025-200662 xenograft tumor model in vivo**

| **Group** | **Treatment** | **N** | **Tumor volume (mm³) (Mean ± SEM)** | | **TGI% Day 21** | **T/C% Day 21** | ***p* value (VS. Vehicle)** | **Complete tumor regression ^{a}(%)** |
|---|---|---|---|---|---|---|---|---|
| | | | **Day 0** | **Day 21** | | | | |
| G1 | Vehicle | 8 | 170.25±12.96 | 1261.93±184.86 | -- | -- | -- | 0 (0/8) |
| G2 | ASK-EGFR, 20 mg/kg | 8 | 169.31±14.43 | 1239.58±146.6 | 1.96 | 98.77 | 0.906 | 0 (0/8) |
| G3 | ASK-MET, 1 mg/kg | 8 | 168.78±15.38 | 116.01±35.11*** | 131.27 | 9.27 | <0.001 | 12.5 (1/8) |
| G4 | ASK-MET, 3 mg/kg | 8 | 170.65±12.40 | 3.54±2.35*** | 197.93 | 0.28 | <0.001 | 75 (6/8) |
| G5 | ASK-MET, 1 mg/kg +ASK-EGFR, 20 mg/kg | 8 | 169.49±13.90 | 32.67±11.56*** | 180.72 | 2.60 | <0.001 | 12.5 (1/8) |
| G6 | ASK-MET, 3 mg/kg +ASK-EGFR, 20 mg/kg | 8 | 170.48±13.92 | 0.00±0.00*** | 200.00 | 0.00 | <0.001 | 100 (8/8) |
| G7 | RefT, 3 mg/kg +ASK-EGFR, 20 mg/kg | 8 | 170.03±13.15 | 100.67±24.39*** | 140.79 | 7.99 | <0.001 | 0 (0/8) |

| | | | | | | | | |
|---|---|---|---|---|---|---|---|---|
| Note: N: Number of animals in each treatment group; Compared with the vehicle control group, * *p<* 0.05, *** p<* 0.01, **** p<* 0.001, *p<* 0.05 and T/C%<40% indicate a significant difference. a: the rate of mice with complete tumor regression after treatment in each treatment group observed by naked eyes on day 21. | | | | | | | | |

**Table 11. Statistical analysis results of combined treatment groups and single drug treatment groups (day 21)**

| **Group** | **Treatment** | **N** | **Tumor volume (mm³) (Mean ± SEM)** | | ***p* value (VS. ASK-EGFR, 20 mg/kg)** | ***p* value (VS. ASK-MET, 1 mg/kg)** | ***p* value (VS. ASK-MET, 3 mg/kg)** | **Q value** |
|---|---|---|---|---|---|---|---|---|
| | | | **Day 0** | **Day 21** | | | | |
| G1 | Vehicle | 8 | 170.25±12.96 | 1261.93±184.86 | -- | -- | -- | -- |
| G2 | ASK-EGFR, 20 mg/kg | 8 | 169.31±14.43 | 1239.58±146.6 | -- | -- | -- | -- |
| G3 | ASK-MET, 1 mg/kg | 8 | 168.78±15.38 | 116.01±35.11 | -- | -- | -- | -- |
| G4 | ASK-MET, 3 mg/kg | 8 | 170.65±12.40 | 3.54±2.35 | -- | -- | -- | -- |
| G5 | ASK-MET, 1 mg/kg +ASK-EGFR, 20 mg/kg | 8 | 169.49±13.90 | 32.67±11.56^{###▲} | <0.001 | 0.046 | -- | 1.38 |
| G6 | ASK-MET, 3 mg/kg +ASK-EGFR, 20 mg/kg | 8 | 170.48±13.92 | 0.00±0.00^{###▼} | <0.001 | -- | 0.044 | 1.02 |
| G7 | RefT, 3 mg/kg +ASK-EGFR, 20 mg/kg | 8 | 170.03±13.15 | 100.67±24.39^{###} | <0.001 | -- | -- | -- |

| | | | | | | | | |
|---|---|---|---|---|---|---|---|---|
| Note: N: Number of animals in each treatment group; compared with ASK-EGFR_20 mg/kg treatment group, ^{#} *p<* 0.05, *^{##} p<* 0.01, ^{###} *p<* 0.001, and *p<* 0.05 indicate a significant difference; compared with ASK-MET_1 mg/kg treatment group, ^{▲}*p<* 0.05, and *p<* 0.05 indicate a significant difference; and compared with ASK-MET_ 3 mg/kg treatment group, ^{▼}*p<* 0.05, and *p<* 0.05 indicate a significant difference. | | | | | | | | |

**Table 12. Tumor weight (g) (Mean ± SEM)**

| **Group** | **Treatment** | **N** | **Day 21 after treatment (g)** | **T/C (%)** | ***p* value** |
|---|---|---|---|---|---|
| G1 | Vehicle | 8 | 1.28±0.19 | -- | -- |
| G2 | ASK-EGFR, 20 mg/kg | 8 | 1.19±0.16 | 93.41 | 0.688 |
| G3 | ASK-MET, 1 mg/kg | 8 | 0.12±0.04 | 9.18 | <0.001 |
| G4 | ASK-MET, 3 mg/kg | 8 | 0.01±0.00 | 0.39 | <0.001 |
| G5 | ASK-MET, 1 mg/kg +ASK-EGFR, 20 mg/kg | 8 | 0.04±0.01 | 3.25 | <0.001 |
| G6 | ASK-MET, 3 mg/kg +ASK-EGFR, 20 mg/kg | 8 | 0.00±0.00 | 0.00 | <0.001 |
| G7 | RefT, 3 mg/kg +ASK-EGFR, 20 mg/kg | 8 | 0.12±0.03 | 9.68 | <0.001 |

| | | | | | |
|---|---|---|---|---|---|
| Note: N: Number of animals in each treatment group; compared with the vehicle control group, * *p<* 0.05, ** *p<* 0.01, *** *p<* 0.001, *p<* 0.05 and T/C%<40% indicate a significant difference. | | | | | |

### 7.2 Weight change of mice

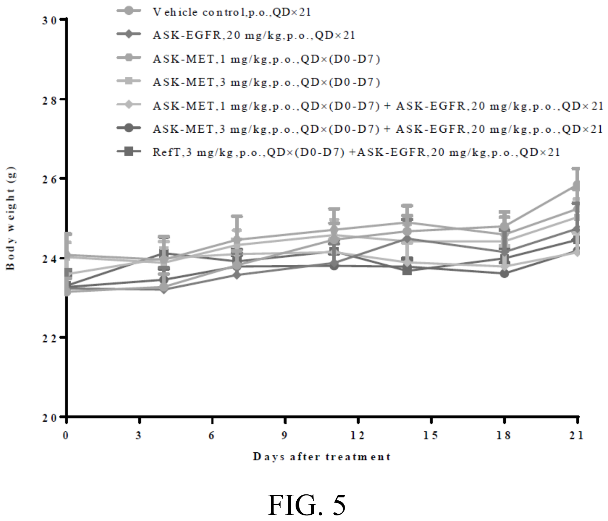

The results of weight change and relative weight change in each treatment group are shown in FIG. 5 and Table 13. During the experiment, all the mice in the treatment groups do not have obvious weight loss and other abnormal symptoms, which indicates that the tumor-bearing mice have good tolerance to the test drugs.

**Table 13. Changes of body weight in each treatment group and control group in human lung cancer LD1-0025-200662 xenograft tumor model**

| **Group** | **Number of animals at the start/end of administration** | **Average weight (g) (Mean ± SEM)** | | **Rate of weight change (%) Day 21** |
|---|---|---|---|---|
| | | **Day 0** | **Day 21** | |
| G1 | 8/8 | 23.15±0.35 | 25.85±0.40 | 11.78±2 |
| G2 | 8/8 | 23.24±0.47 | 24.75±0.64 | 6.45±1.28 |
| G3 | 8/8 | 24.08±0.53 | 25.24±0.50 | 4.88±0.71 |
| G4 | 8/8 | 24.02±0.37 | 25.03±0.45 | 4.19±1.08 |
| G5 | 8/8 | 23.60±0.44 | 24.14±0.50 | 2.37±1.42 |
| G6 | 8/8 | 23.27±0.37 | 24.19±0.27 | 4.03±1.15 |
| G7 | 8/8 | 23.30±0.27 | 24.46±0.23 | 5.06±1.56 |

### 8. Experimental conclusion and discussion

According to the above experimental results, after treatment, the ASK-MET_1 mg/kg treatment group, ASK-MET_3 mg/kg treatment group, ASK-MET_1 mg/kg+ASK-EGFR_20 mg/kg treatment group, ASK-MET_3 mg/kg+ASK-EGFR_20 mg/kg treatment group and RefT_3 mg/kg+ASK-EGFR_20 mg/kg treatment group all show statistically significant inhibition on the growth of subcutaneous xenograft tumor in mice inoculated with LD1-0025-200662 human lung cancer tissue. Moreover, the ASK-MET_1 mg/kg treatment group, ASK-MET_3 mg/kg treatment group, ASK-MET_1 mg/kg+ASK-EGFR_20 mg/kg treatment group and ASK-MET_3 mg/kg+ASK-EGFR_20 mg/kg treatment group all show varying degrees of complete tumor regression of tumor-bearing mice. Moreover, the ASK-MET_1 mg/kg+ASK-EGFR_20 mg/kg combined treatment group and ASK-MET_3 mg/kg+ASK-EGFR_20 mg/kg combined treatment group show an obviously better anti-tumor effect than that of the corresponding single drug treatment groups, and show an obvious synergistic effect. During the whole treatment process, there is no obvious abnormality in the weight and behavior of tumor-bearing mice, which indicates that the tumor-bearing mice have good tolerance to the test drugs.

### Example 3. Open, multi-center, phase I clinical trial on the safety, tolerability, pharmacokinetics and preliminary effectiveness of ASKC202 tablet in combination with ASK120067 tablet in patients with advanced solid tumors

Clinical trial registration number: CTR20220697; ASKC202 is ASK-MET in Examples 1 and 2, and ASK120067 is ASK-EGFR in Examples 1 and 2.

**Table 14. Test drugs and dosing regimen**

| **No.** | **Drug name** | **Specification** | **Dosage and Administration** | **Schedule of administration** |
|---|---|---|---|---|
| 1 | ASKC202 tablet | 50 mg/tablet | 1 tablet each day | Single administration: only 1 oral administration during Cycle0; |
| | | | | Consecutive administration: once a day during Cycle1, for 21 consecutive days, followed by repeatedly cyclical administration with each 21 days as an administration cycle (Cycle2-CycleN), until disease progress or intolerable toxicity occurs |
| 2 | ASKC202 tablet | 200 mg/tablet | 1 tablet each day | Single administration: only 1 oral administration during Cycle0; |
| | | | | Consecutive administration: once a day during Cycle1, for 21 consecutive days, followed by repeatedly cyclical administration with each 21 days as an administration cycle (Cycle2-CycleN), until disease progress or intolerable toxicity occurs |
| 3 | ASK120067 tablet | 80 mg/tablet | 1 tablet each time, twice a day | Consecutive administration: twice a day during Cycle 1, for 21 consecutive days, followed by repeatedly cyclical administration with each 21 days as an administration cycle (Cycle2-CycleN), until disease progress or intolerable toxicity occurs |

In the 1st treatment cycle, the AE of 4 subjects recruited in this study was mainly abnormal laboratory examinations, and obvious anti-tumor activity was initially observed in 2 subjects in the first tumor evaluation. Therefore, the combination of ASKC202 tablet and ASK120067 tablet has broad prospects in the treatment of locally advanced or metastatic non-small cell lung cancer.

All documents mentioned in the present invention are cited as references in this invention, as if each document is individually cited as a reference. Moreover, it should be understood that after reading the above contents of the present invention, various changes or modifications can be made by those skilled in the art to the present invention, which also fall within the scope defined by the appended claims of the present invention.

## Claims

1. A drug combination for treating tumor diseases, comprising (i) an EGFR kinase inhibitor; and (ii) a c-Met kinase inhibitor, wherein the EGFR kinase inhibitor is selected from a compound of Formula (I) or a pharmaceutically acceptable salt thereof, and the c-Met kinase inhibitor is selected from a compound of Formula (II) or a pharmaceutically acceptable salt thereof,

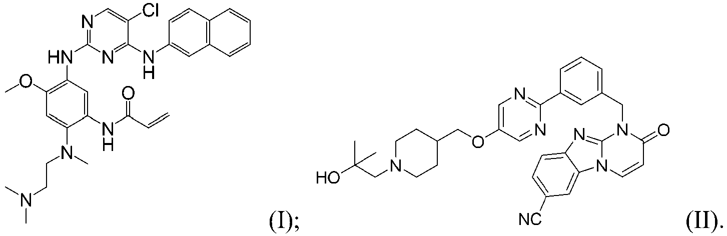

2. Use of a combination of a compound of Formula (I) or a pharmaceutically acceptable salt thereof and a compound of Formula (II) or a pharmaceutically acceptable salt thereof in the preparation of a drug for treating tumor diseases;

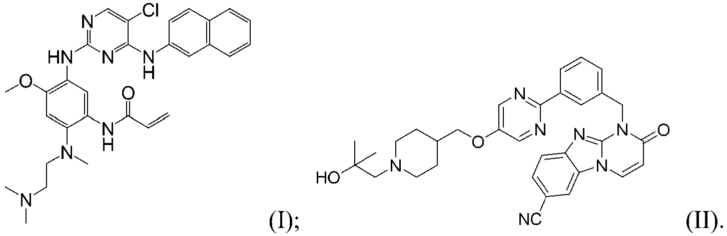

3. The drug combination according to claim 1 or the use according to claim 2, wherein the tumor diseases are selected from breast cancer, ovarian cancer, prostate cancer, melanoma, brain tumor, esophageal carcinoma, gastric cancer, liver cancer, pancreatic cancer, colorectal cancer, lung cancer, kidney cancer, skin cancer, glioblastoma, neuroblastoma, sarcoma, liposarcoma, osteochondroma, osteoma, osteosarcoma, seminoma, testicular tumor, cervical cancer, head and neck tumor, multiple myeloma, malignant lymphoma, polycythemia vera, leukemia, thyroid neoplasm, ureteral tumor, bladder cancer, gallbladder cancer, bile duct carcinoma or chorionepithilioma, preferably lung cancer, and further preferably non-small cell lung cancer.

4. The drug combination according to claim 1 or the use according to claim 2, wherein the tumor diseases are tumor diseases in patients resistant to EGFR kinase inhibitors; and preferably non-small cell lung cancer resistant to EGFR kinase inhibitors.

5. The drug combination according to claim 1 or the use according to claim 2, wherein the tumor diseases are tumor diseases associated with EGFR mutation and/or MET alteration; preferably non-small cell lung cancers associated with EGFR mutation and/or MET alteration; and further preferably non-small cell lung cancers associated with one or more of EGFR 19del, MET alteration, EGFR L858R, and EGFR T790M.

6. The drug combination according to claim 1 or the use according to claim 2, wherein the tumor diseases are non-small cell lung cancer that has disease progression after treatment with EGFR kinase inhibitors previously and is associated with MET alteration.

7. The drug combination according to claim 1 or the use according to claim 2, wherein
the single dosage of the compound of Formula (I) or the pharmaceutically acceptable salt thereof is 40-320 mg, preferably 60-240 mg, and further preferably 80-160 mg;
the frequency of administration of the compound of Formula (I) or the pharmaceutically acceptable salt thereof is once a day, twice a day, three times a day or once every two days;
the single dosage of the compound of Formula (II) or the pharmaceutically acceptable salt thereof is 40-640 mg, preferably 40-320 mg, and further preferably 60-320 mg; and
the frequency of administration of the compound of Formula (II) or the pharmaceutically acceptable salt thereof is once a day, twice a day, three times a day or once every two days.

8. The drug combination according to claim 1 or the use according to claim 2, wherein the compound of Formula (I) or the pharmaceutically acceptable salt thereof and the compound of Formula (II) or the pharmaceutically acceptable salt thereof are administered simultaneously or at different times.

9. A pharmaceutical preparation, comprising the drug combination according to claim 1 and one or more pharmaceutically acceptable carriers.
